# DEMANDE DE BREVET EUROPEEN

(11) **EP 2 269 588 A1**
(43) Date de publication de la demande: **05.01.2011**
(21) Numéro de dépôt: 10182581.8
(22) Date de dépôt: 25.04.2002
(51) Int. Cl.: A61K 9/22, A61K 31/465

(54) **Pastille medicamenteuse a liberation lente du principe actif**

(30) Priorité: 25.04.2001 FR 0105554
(62) Demande divisionnaire de: 10154760.2
(71) Demandeur: PIERRE FABRE MEDICAMENT, 92100 Boulogne-Billancourt (FR)
(72) Inventeur: Dupinay, Pierre, 31640, TOUTENS (FR); Torres, Robert, 31520, RAMONVILLE SAINT AGNE (FR); Capon, Christine, 31520, RAMONVILLE SAINT AGNE (FR)
(74) Mandataire: Ahner, Francis

(57) **Abrégé**

Pastille médicamenteuse à sucer de sucre cuit, de consistance solide, destinée à se dissoudre dans la cavité buccale, comprenant au moins un principe actif, **caractérisée en ce qu**'elle comprend en outre au moins un agent matriciel permettant de ralentir la libération du ou des principes actifs qui restent alors au contact prolongé de la sphère buccopharyngée, le temps de dissolution dans la cavité buccale étant d'au moins 15 minutes.

## Description

L'invention concerne des pastilles médicamenteuses à sucer, de consistance solide destinées à se dissoudre dans la cavité buccale, comprenant un agent matriciel ralentissant la libération du ou des principes actifs dans la sphère buccopharyngée. L'invention concerne également un procès de fabrication d'une telle pastille.

On connaît déjà des pastilles à sucer médicamenteuses qui sont des préparations à base de substances sucrantes, de consistance solide, destinées à se dissoudre dans la cavité buccale.

Elles se présentent généralement sous des formes variées : sphères, cylindres, carrés ou toute autre forme polygonale.

Ces pastilles encore appelées sucres cuits sont préparées à partir d'un sirop de substance diluante sucrée porté à ébullition, puis cuit à une température plus élevée, typiquement de 100°C à 160°C. A cette base sucrée sont ajoutées des substances auxiliaires telles qu'édulcorants, antioxydants, colorants, arômes, et le ou les principes actifs.

En fin de cuisson le ou les principes actifs sont ajoutés à la masse dans un mélangeur ainsi que les substances auxiliaires.

La masse ainsi préparée est pétrie sur une surface froide appropriée, puis roulée et filée, pour être ensuite pressée ou découpée en pastilles à la forme désirée.

Ces pastilles à sucer médicamenteuses de sucre cuit sont essentiellement destinées, du fait de leur lieu de dissolution, aux traitements locaux de la sphère buccale et oropharyngienne mais aussi aux principes actifs absorbés par voie perlinguale.

De ce fait il est nécessaire que les principes actifs choisis pour ces modes d'action se libèrent progressivement pour rester au contact de la sphère buccopharyngée le plus longtemps possible, tout en évitant un passage rapide et massif dans le tractus digestif, ce qui aurait pour conséquence de les rendre inefficaces sur le lieu d'application ou la voie d'absorption choisis.

Or, les pastilles médicamenteuses de sucre cuit actuelles se dissolvent très rapidement en bouche, entre 5 et 10 minutes maximum, libérant ainsi trop vite le ou les principes actifs qu'elles contiennent et qui de ce fait sont aussitôt déglutis et absorbés au niveau du tractus digestif.

La présente invention propose de nouvelles pastilles médicamenteuses de sucre cuit qui permettent de pallier cet inconvénient de l'art antérieur.

A cet effet l'invention concerne selon un premier aspect une pastille médicamenteuse à sucer de sucre cuit, de consistance solide, destinée à se dissoudre dans la cavité buccale, comprenant au moins un principe actif, la pastille comprenant en outre au moins un agent matriciel permettant de ralentir la libération du ou des principes actifs qui restent alors au contact prolongé de la sphère buccopharyngée.

L'agent matriciel est un agent apte à ralentir la dissolution du sucre cuit en bouche. Selon un mode de réalisation, l'agent matriciel confère en outre à la pastille une résistance accrue, durable même au contact de la salive de sorte que le patient ne peut croquer cette pastille et en avaler des morceaux.

Le temps de dissolution dans la cavité buccale de la pastille est d'au moins 15 minutes, et typiquement de 25 à 35 minutes, de préférence 30 minutes.

L'agent matriciel est typiquement choisi dans le groupe constitué par les polysaccharides non cellulosiques, les dérivés cellulosiques, les polymères de l'acide acrylique, les corps gras, le polyvinylpyrrolidone, ces substances étant utilisées seules ou en mélange et représentant 1 à 10% en poids de la pastille, typiquement 1 à 5%. Selon une réalisation, l'agent matriciel est choisi dans le groupe constitué par : la gomme guar, la gomme de caroube, l'alginate de sodium et de potassium, l'agar-agar, le carragaheen, la gomme arabique, la gomme sterculia, la gomme adragante.

Selon une réalisation, l'agent matriciel est choisi dans le groupe constitué par l'hydroxypropylcellulose, l'hydroxypropylméthylcellulose, l'hydroxyéthylcellulose, l'éthylcellulose.

Selon une réalisation, le polymère de l'acide acrylique est un carbomère ou un polyméthacrylate, copolymère d'acétate de vinyle.

Selon une réalisation, le corps gras est choisi dans le groupe constitué par les cires, les gélucires, le glycéryl béhénate, le glycéryl palmitostéarate.

Selon une réalisation, l'agent matriciel est le polyvinylpyrrolidone.

La pastille comprend en outre un excipient sucré majeur, ou diluant, choisi parmi le saccharose, le fructose, le lactose, le sorbitol, le mannitol, le lactitol, le glucose, le maltitol, l'isomalt, le polydextrose, les maltodextrines, utilisés seuls ou en mélange représentant 80 à 99% en poids de la pastille.

Selon une réalisation, la pastille comprend en outre au moins une substance auxiliaire choisie parmi les édulcorants, les antioxydants, les colorants, les arômes.

Selon une réalisation préférée, l'invention concerne une pastille comprenant comme principe actif la nicotine.

Selon une réalisation préférée, la pastille comprend de l'isomalt, du méthocel (hydroxypropylcellulose), de la nicotine et un agent sucrant à fort pouvoir sucrant, notamment de l'aspartam.

Selon un autre aspect l'invention a pour objet un procédé de fabrication d'une pastille médicamenteuse décrite précédemment, comprenant successivement :
- une étape d'ébullition d'un sirop de substance diluante sucrée;
- une étape de cuisson à une température plus élevée, de l'ordre de 100°C à 160°C;
- une étape de mélange avec incorporation du ou des principes actifs et de la ou des substances auxiliaires;
- une étape de fabrication de la pastille de sucre suit ;
   l'agent matriciel étant incorporé soit au cours de l'étape d'ébullition, soit au cours de l'étape de cuisson, soit lors du mélange.

D'autres aspects et avantages de l'invention apparaîtront lors de la description détaillée qui suit.

L'agent matriciel permet de ralentir la dissolution du sucre cuit en bouche et par la même la libération du ou des principes actifs sur le site d'action.

Par ailleurs fait inattendu, contrairement à ce que l'on aurait pu penser, l'agent matriciel confère à la pastille de sucre cuit une résistance importante, durable même au contact de la salive, supérieure à celle habituellement obtenue sur ce type de préparation, de sorte qu'il devient impossible au patient de croquer cette pastille et d'en avaler les morceaux.

Ce phénomène complémentaire est particulièrement favorable à l'effet prolongé recherché sur le site d'action, puisque le patient se trouve dans l'obligation de laisser fondre la pastille en bouche jusqu'à sa complète dissolution.

Le temps de dissolution dans la cavité buccale, obtenu avec cette nouvelle forme de pastilles de sucre cuit, peut atteindre 30 minutes. Ce temps peut être modulé en fonction du choix du ou des agents sucrants et du ou des agents matriciels.

Ces nouvelles pastilles de sucre cuit peuvent présenter toutes les formes géométriques habituelles déjà citées auparavant.

Elles sont composées en forte proportion d'une substance diluante sucrée ou excipient constituant la base de la préparation, qui peut être : le Saccharose, le Fructose, le Lactose, le Sorbitol, le Mannitol, le Lactitol, le Glucose, le Maltitol, l'Isomalt, le Polydextrose, les Maltodextrines....

Parmi les polysaccharides non cellulosiques utilisables on citera notamment : gomme guar, gomme de caroube, alginate de sodium et de potassium, agar-agar, carragaheen, gomme arabique, gomme sterculia, gomme adragante, gomme xanthane, gomme tragacanthe, gomme karaya.

Parmi les dérivés cellulosiques utilisables, on préfèrera les dérivés : hydroxypropylcellulose, hydroxypropylméthylcellulose, hydroxyéthylcellulose, éthylcellulose. Les hydroxy-éthylcelluloses sont par exemple décrites dans *"*Handbook of water-soluble gums and resins", ed.R .L.Davidson, pub.McGraw-Hill (1980). On pourra aussi utiliser notamment les dérivés : carboxyméthylcellulose (CMC), méthylcellulose (MC), éthylhydroxyéthylcellulose (EHEC), hydroxyéthylméthylcellulose (HEMC), hydroxyéthylcellulose modifié (HMHEC), éthylhydroxyéthylcellulose modifié (HMEHEC), carboxyméthylhydroxyéthylcellulose (CMHEC).

Les substances constituant l'agent matriciel peuvent être utilisées seules ou en mélange. Leurs choix, leurs concentrations seront fonction de la ou des substances sucrantes utilisées, du ou des principes actifs retenus et de leur activité, du temps de dissolution en bouche et de la texture du sucre cuit souhaités.

L'excipient sucré majeur, ou diluant, est choisi parmi les substances sucrées précédemment citées utilisées seules ou en association, là aussi comme précédemment, en fonction du résultat souhaité.

Les substances auxiliaires sont celles habituellement mises en oeuvre : édulcorants, antioxydants, colorants, arômes....

Le procédé de fabrication selon l'invention comprend quatre étapes : une étape d'ébullition, une étape de cuisson, une étape de mélange, une étape de fabrication des pastilles de sucre cuit.

Le procédé de fabrication est conforme aux exigences pharmaceutiques actuelles. Le principe actif peut être par exemple : nicotine, digluconate de chlorhexidine, tetracaïne, cetylpiridinium, extrait de végétaux.

L'agent matriciel peut être incorporé soit au cours de l'étape d'ébullition, soit au cours de l'étape de cuisson, soit lors du mélange.

Le choix de l'étape d'incorporation de l'agent matriciel sera fonction des caractéristiques finales des pastilles de sucre cuit souhaitées.

Les pastilles ainsi obtenues sont très différentes d'autres formes galéniques telles que des comprimés susceptibles de contenir certains des polysaccharides non cellulosiques et des dérivés cellulosiques cités précédemment.

En effet, la forme galénique pastille est bien spécifique, ayant fait l'objet d'une monographie à la Pharmacopée Française Xème Edition (juillet 1987). Les pastilles sont des saccharoïdes de consistance solide destinées à se désagréger lentement dans la cavité buccale. Elles se présentent notamment sous une forme hémisphérique et pèsent généralement entre et 1g et 3 g. Elles sont généralement composées de saccharose en forte proportion, d'une substance agglomérante telle que gomme arabique ou gomme adragante, d'un ou plusieurs principes actifs et éventuellement de substances auxiliaires (colorants, aromatisants ...). Les pastilles sont préparées en réalisant d'abord une pâte avec une partie de saccharose. Cette pâte est chauffée jusqu'à légère ébullition. Le reste du saccharose, le ou les principes actifs ainsi que les substances auxiliaires sont alors ajoutés. Après homogénéisation on fait tomber la masse ainsi préparée goutte à goutte sur une plaque froide ou on l'injecte dans un moule approprié. Ainsi la pastille diffère nettement de comprimés :
- par son mode d'utilisation
- par sa composition, et notamment les excipients utilisés (saccharose, isomalt, maltitol, fructose, glucose) et leur concentration élevée de l'ordre de 80 à 99% de la formule
- par son mode de préparation spécifique, totalement différent des comprimés, et nécessitant un appareillage spécifique :
   o cuisson du mélange sous vide d'où le terme de « sucre cuit »
   o mélange et refroidissement de la pâte obtenue sur une table froide munie d'un pétrisseur
   o extrusion de la masse
   o pressage sur presse totalement différente d'une comprimeuse

En particulier le pourcentage d'agent matriciel dans la pastille est faible, de l'ordre de 1 à 10%, de préférence de 1 à 5%.

On présente ci-après quelques exemples de pastilles selon l'invention.

### Exemple 1

| | |
|---|---|
| Digluconate de chlorhexidine | 3,000 mg |
| Tetracaïne chlorhydrate | 0,200 mg |
| Acide ascorbique | 52,500 mg |
| Hydroxypropyl methylcellulose | 100,000 mg |
| Isomalt | 2 317,1875 mg |
| Glycyrrhizinate d'ammonium | 5,000 mg |
| Aspartam | 1,000 mg |
| Arôme | QS |
| Eau | QS |

### Exemple 2

| | |
|---|---|
| Digluconate de chlorhexidine | 3,000 mg |
| Acide ascorbique | 52,500 mg |
| Hydroxypropyl methylcellulose | 50,000 mg |
| Maltitol | 2 425,000 mg |
| Erythrosine | 0,250 mg |
| Arômes | QS |
| Eau | QS |

### Exemple 3

| | |
|---|---|
| Cetylpyridinium | 2,500 mg |
| Benzocaïne | 2,000 mg |
| Vitamine C | 52,500 mg |
| Hydroxypropyl methylcellulose | 100,000 mg |
| Isomalt | 2 425,000 mg |
| Arômes | QS |
| Eau | QS |

### Exemple 4

| | |
|---|---|
| Extrait d'erysimum | 15,000 mg |
| Carraghenate | 100,000 mg |
| Saccharose | 1 471,250 mg |
| Glucose | 1 000,000 mg |
| Glycyrrhizinate d'ammonium | 1,000 mg |
| Arôme | QS |
| Eau | QS |

### Exemple 5

| | |
|---|---|
| Nicotine | 1,000 ou 2,000 mg |
| Hydroxypropyl methylcellulose | 100,000 mg |
| Cyclodextrine | 50,000 mg |
| Isomalt | 2 306,083 mg |
| Aspartam | 0,750 mg |
| Arôme | QS |
| Eau | QS |

### Exemple 6

| | |
|---|---|
| Chlorhydrate d'amyleïne | 2,000 mg |
| Chlorhydrate de cetylpyridinium | 1,000 mg |
| Polyvinylpyrrolidone | 100,000 mg |
| Saccharose | 1577,000 mg |
| Glucose | 820,000 mg |
| Arôme | QS |
| Colorant | QS |
| Eau | QS |

### Exemple 7

| | |
|---|---|
| Dextromethorphane bromhydrate | 20,000 mg |
| Acide citrique | 37,000 mg |
| Polyméthacrylate | 50,000 mg |
| Saccharose | 1 256,000 mg |
| Glucose | 1 111,000 mg |
| Arôme | QS |
| Eau | QS |

### Exemple 8

### Formule unitaire

| Matières Premières | Quantité |
|---|---|
| Isomalt Type M | 2350,10 mg |
| Methocel K 15 M | 100,00 |
| Nicotine Polacrilex titré 18% | 11,835 |
| Aspartam | 0,750 |
| Acesulfam K | 1,250 |
| Arôme Peppermint 13-571-016 | 16,00 |
| Arôme Peppermint 13-627-517 | 20,00 |
| Total | 2500 mg |

### Exemple 9

### Formule unitaire

| Matières Premières | Quantité |
|---|---|
| Isomalt Type M | 2338,76 mg |
| Methocel K 15 M | 100,00 |
| Nicotine Polacrilex titré 18% | 11,835 |
| Aspartam | 0,900 |
| Acesulfam K | 1,500 |
| Arôme Peppermint 13-571-016 | 8,00 |
| Extrait sec de réglisse déglycyrrhiziné | 37,50 |
| Glycyrrhizinate d'ammonium | 1,500 |
| Total | 2500 mg |

Ainsi, la pastille des exemples 8 et 9 comprend 2,1303 g de nicotine.

La fabrication de ces pastilles est réalisée à chaud. L'agent matriciel est incorporé dans la base sucrante à une température de l'ordre de 70 à 95°C (étape d'ébullition), de préférence à 90°C. L'ensemble est ensuite chauffé à environ 130°C au moment de la cuisson. La nicotine est alors ajoutée (étape du mélange) dans la masse à une température de l'ordre de 110 à 130°C, de préférence 120°C.

La nicotine Polacrilex est un complexe de nicotine C₁₀H₁₄N₂ avec une résine échangeuse d'ions (catiorésine carboxylate); ce complexe à pour formule :C₁₀H₁₄N₂(C₁₈H₂₂O₄)ₙ

## Revendications

1. Pastille médicamenteuse à sucer de sucre cuit, de consistance solide, destinée à se dissoudre dans la cavité buccale, comprenant au moins un principe actif, **caractérisée en ce qu'**elle comprend en outre au moins un agent matriciel permettant de ralentir la libération du ou des principes actifs qui restent alors au contact prolongé de la sphère buccopharyngée, le temps de dissolution dans la cavité buccale étant d'au moins 15 minutes.

2. Pastille selon la revendication 1 **caractérisée en ce que** l'agent matriciel est un agent apte à ralentir la dissolution du sucre cuit en bouche.

3. Pastille selon l'une quelconque des revendications 1 ou 2 **caractérisée en ce que** l'agent matriciel confère en outre à la pastille une résistance accrue, durable même au contact de la salive de sorte que le patient ne peut croquer cette pastille et en avaler des morceaux.

4. Pastille selon l'une quelconque des revendications 1 à 3 **caractérisée en ce que** le temps de dissolution dans la cavité buccale de la pastille est de 25 à 35 minutes, de préférence 30 minutes.

5. Pastille selon l'une quelconque des revendications 1 à 4 **caractérisée en ce que** l'agent matriciel est choisi dans le groupe constitué par les polysaccharides non cellulosiques, les dérivés cellulosiques, les polymères de l'acide acrylique, les corps gras, le polyvinylpyrrolidone, ces substances étant utilisées seules ou en mélange et représentant 1 à 10% en poids de la pastille, typiquement 1 à 5%.

6. Pastille selon l'une quelconque des revendications 1 à 5 **caractérisée en ce que** l'agent matriciel est choisi dans le groupe constitué par : la gomme guar, la gomme de caroube, l'alginate de sodium et de potassium, l'agar-agar, le carragaheen, la gomme arabique, la gomme sterculia, la gomme adragante.

7. Pastille selon l'une quelconque des revendications 1 à 5 **caractérisée en ce que** l'agent matriciel est choisi dans le groupe constitué par l'hydroxypropylcellulose, l'hydroxypropylméthylcellulose, l'hydroxyéthylcellulose, l'éthylcellulose.

8. Pastille selon l'une quelconque des revendications 1 à 5 **caractérisée en ce que** le polymère de l'acide acrylique est un carbomère, un polyméthacrylate ou un copolymère d'acétate de vinyle.

9. Pastille selon l'une quelconque des revendications 1 à 5 **caractérisée en ce que** le corps gras est choisi dans le groupe constitué par les cires, les gélucires, le glycéryl béhénate, le glycéryl palmitostéarate.

10. Pastille selon l'une quelconque des revendications 1 à 5 **caractérisée en ce que** l'agent matriciel est le polyvinylpyrrolidone.

11. Pastille selon l'une quelconque des revendications 1 à 10 **caractérisée en ce qu'**elle comprend en outre un excipient sucré majeur, ou diluant, choisi parmi le saccharose, le fructose, le lactose, le sorbitol, le mannitol, le lactitol, le glucose, le maltitol, l'isomalt, le polydextrose, les maltodextrines, utilisés seuls ou en mélange, représentant 80 à 99% en poids de la pastille.

12. Pastille selon l'une quelconque des revendications 1 à 11 **caractérisée en ce qu'**elle comprend en outre au moins une substance auxiliaire choisie parmi les édulcorants, les antioxydants, les colorants, les arômes.

13. Pastille selon l'une quelconque des revendications 1 à 11 **caractérisée en ce qu'**elle comprend comme principe actif la nicotine.

14. Pastille selon la revendication 13 **caractérisée en ce qu'**elle comprend de l'isomalt, du méthocel, de la nicotine, de l'aspartam.

15. Procédé de fabrication d'une pastille médicamenteuse selon l'une quelconque des revendications 1 à 13 **caractérisé en ce qu'**il comprend successivement :
- une étape d'ébullition d'un sirop de substance diluante sucrée
- une étape de cuisson à une température plus élevée, de l'ordre de 100°C à 160°C
- une étape de mélange avec incorporation du ou des principes actifs et de la ou des substances auxiliaires
- une étape de fabrication de la pastille ;
l'agent matriciel étant incorporé soit au cours de l'étape d'ébullition, soit au cours de l'étape de cuisson, soit lors du mélange.
